# FASCICULE DE BREVET EUROPEEN

(11) **EP 2 550 350 B1**
(45) Date de publication et mention de la délivrance du brevet: **28.01.2015**
(21) Numéro de dépôt: 11715984.8
(22) Date de dépôt: 24.03.2011
(51) Int. Cl.: C12M 1/22, B01L 3/00

(54) **BOITE DE PETRI COMPORTANT DES MOYENS DE RETENUE DU MILIEU DE CULTURE GELOSE**
PETRISCHALE MIT EINEM MITTEL ZUM HALTEN EINES AGAR-KULTURMEDIUMS
PETRI DISH COMPRISING MEANS FOR HOLDING AGAR CULTURE MEDIUM

(30) Priorité: 24.03.2010 FR 1052121
(43) Date de publication de la demande: 30.01.2013
(73) Titulaire: Biomérieux, 69280 Marcy L'etoile (FR)
(72) Inventeur: COLIN, Bruno, F-69280 Marcy l'Etoile (FR); SIMON, Nathalie, F-69290 Grézieu La Varenne (FR)
(86) Numéro de dépôt international: PCT/FR2011/050625
(87) Numéro de publication internationale: WO 2011/117545

(56) Documents cités:
- JP-A- 06 032 345
- JP-A- 10 080 268
- JP-A- 2001 309 777
- US-A1- 2006 172 412
- US-B2- 6 969 607

## Description

Le domaine technique de la présente invention est celui du diagnostic in vitro et plus précisément, celui du diagnostic microbiologique. La présente invention concerne en particulier une boite de Pétri présentant des moyens de retenue du milieu de culture gélosé.

L'utilisation des boites de Pétri dans le domaine microbiologique est connue depuis de nombreuses décennies. En effet, ces boites sont utilisées comme conteneur d'un milieu de culture gélosé, utilisé pour permettre la croissance et la détection des microorganismes. Les boites de Pétri sont généralement circulaires et constituées d'un fond qui contient le milieu de culture et d'un couvercle venant se positionner sur le fond afin d'éviter que le milieu ne soit contaminé par des microorganismes présents dans l'environnement extérieur, tout en permettant le passage de l'air, nécessaire au développement de nombreux microorganismes. Il existe majoritairement deux formes de boites de Pétri :
- la boite de Pétri standard, définie à la norme ISO 24988- 2008, et
- la boite de Pétri dite « contact » qui permet de réaliser des prélèvements de surface et qui présente, à cette fin, une forme particulière telle que le milieu gélosé déborde du fond.

Quel que soit le type de boite, celle-ci joue un rôle primordial dans la qualité et les performances du milieu de culture en tant qu'outil de détection et d'identification de microorganismes. En effet, la boite se doit, en particulier, de présenter des surfaces parfaitement transparentes et dénuées de défauts, de manière à ne pas risquer de fausser la lecture.

Nonobstant la bonne qualité de conception de la boite de Pétri, il peut se produire avec certains milieux de culture gélosés, une rétraction du milieu de culture, une fois coulé dans la boite et solidifié. Ceci se produit généralement après un certain temps de stockage. Cette problématique est bien connue des microbiologistes et est directement liée à la composition du milieu de culture. En effet, certains composés (tels que les agents tensioactifs ou de nature lipidique) présents dans le milieu, diminuent sensiblement la capacité d'adhésion dudit milieu au fond de la boite dans laquelle il est coulé. C'est le cas par exemple du milieu Ottaviani Agosti, utilisé pour la recherche et le dénombrement de *Listeria monocytogenes*, conformément à la norme NF EN ISO 11290. De plus, la forme des boites de Pétri, généralement circulaire, n'est pas propice à assurer un bon maintien en place de la gélose. Il s'ensuit que le milieu gélosé se décolle d'abord de la paroi verticale. Il se produit alors un phénomène de rétraction radiale du milieu gélosé vers le centre de la boite de Pétri. Ledit milieu gélosé finit par se désolidariser complètement de la boite de Pétri, au risque de sortir de la boite lors de la manipulation de cette dernière. Les documents US 2006/172412 A1, JP 10080268 A, JP 2001309777 A et JP 06032345 visent à éviter la rétraction du milieu de culture gélosé.

Un objectif de la présente invention est donc de fournir une boite de Pétri circulaire apte à limiter les effets de rétraction du milieu de culture gélosé contenu, dans ladite boite.

Un autre objectif de la présente invention est de fournir une boite de Pétri présentant des moyens destinés à empêcher la rétraction du milieu de culture gélosé, qui ne gênent pas la lecture de la boite de Pétri.

Ces objectifs parmi d'autres sont atteints par la présente invention qui concerne en premier lieu une boite de Pétri, destinée à recevoir un milieu de culture de microorganismes gélosé, comprenant une base recevant ledit milieu de culture et un couvercle apte à venir se positionner sur la base, ladite base et ledit couvercle étant sensiblement circulaires, ladite boite de Pétri étant caractérisée en ce que la base comprend au moins un moyen destiné à éviter la rétraction du milieu de culture, ledit moyen étant en saillie et positionné en périphérie de la base selon la revendication 1.

Selon un mode de réalisation particulier de la boite de Pétri selon l'invention, le moyen destiné à éviter la rétraction du milieu de culture est constitué par une nervure continue.

Selon un mode de réalisation alternatif, le moyen destiné à éviter la rétraction du milieu de culture est constitué par au moins une nervure discontinue.

Avantageusement, la boite de Pétri selon l'invention comprend des moyens de verrouillage du couvercle sur la base.

Selon un mode préférentiel, les moyens de verrouillage comprennent au moins une patte radiale et au moins une gaine radiale, positionnées respectivement soit sur le couvercle, soit sur la base, et coopérant pour verrouiller la boite de Pétri.

De tels moyens de verrouillage sont décrits, par exemple, dans le brevet US-6,969,607.

Avantageusement, la boite de Pétri selon l'invention comprend entre 2 et 12 nervures discontinues.

De façon particulière, le ou les moyen(s) destiné(s) à éviter la rétraction du milieu de culture présente(nt) une hauteur comprise entre 0,15 et 5 millimètres.

Le ou lesdit(s) moyen(s) destiné(s) à éviter la rétraction du milieu de culture est (sont) situé(s) à une distance comprise entre 0,5 et 15 millimètres du bord vertical circulaire de la base.

Un autre objet de la présente invention concerne un procédé de préparation d'un milieu de culture gélosé comprenant les étapes consistant à :
- Disposer d'une boite de Pétri selon l'invention,
- Répartir un milieu de culture gélosé en fusion à l'intérieur de ladite boite de Pétri, de sorte que ce dernier recouvre l'intégralité du fond de la base,
- Laisser refroidir le milieu de culture gélosé de sorte que le ou les moyens destiné(s) à éviter la rétraction soient ancrés dans le milieu de culture gélosé.

L'invention sera mieux comprise à la lecture de la description détaillée et nullement limitative qui suit, en référence au dessin dans lequel :
La figure 1 représente une vue partielle en perspective de la base de la boite de Pétri selon un premier mode de réalisation.
La figure 2 représente une vue partielle en perspective de la base de la boite de Pétri selon un deuxième mode de réalisation.
La figure 3 représente une vue en coupe transversale de la base de la boite de Pétri selon le premier mode de réalisation.
La figure 4 représente une vue en coupe transversale de la base de la boite de Pétri selon le deuxième mode de réalisation.
La figure 5 représente une vue en coupe transversale de la boite de Pétri selon l'invention, contenant le milieu de culture gélosé.
limitative qui suit, en référence au dessin dans lequel :
La figure 6 représente une vue partielle en perspective de la base de la boite de Pétri selon un troisième mode de réalisation.
La figure 7 représente une vue partielle en perspective de la base de la boite de Pétri selon un quatrième mode de réalisation.

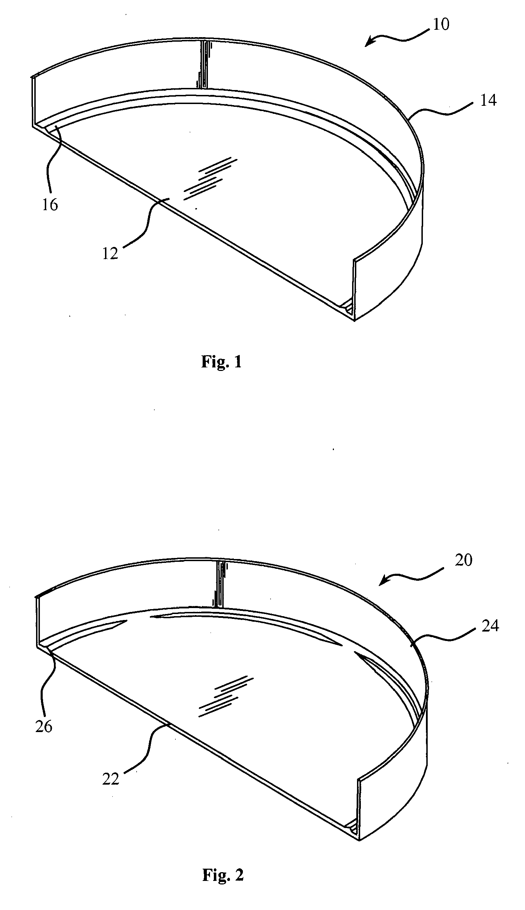

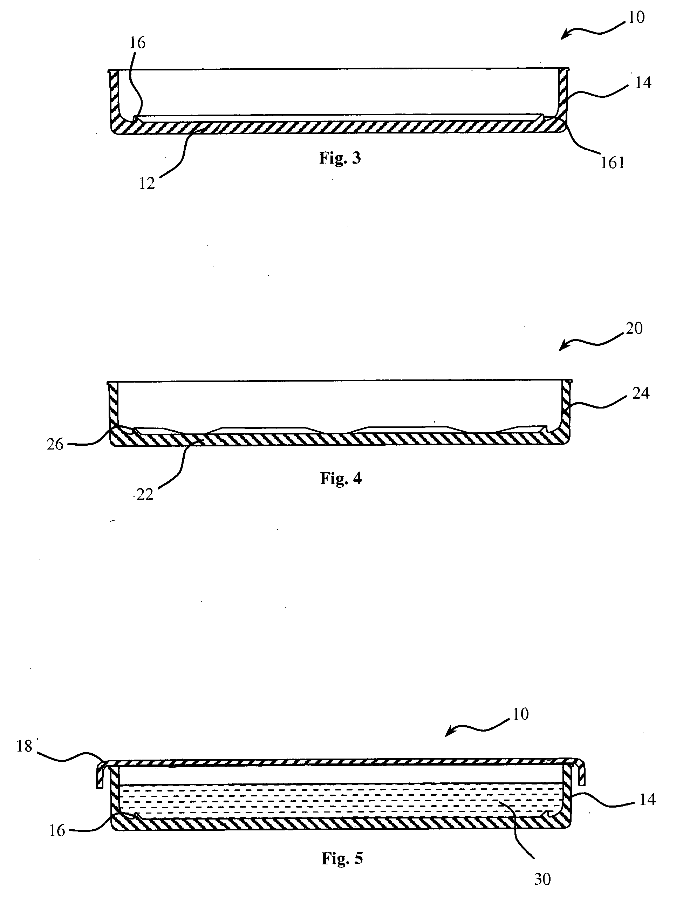

La boite de Pétri selon l'invention présente classiquement une base et un couvercle. Sur la figure 1, on peut observer la base 10 d'une boite de Pétri selon un premier mode de réalisation. Cette base 10 est constituée d'un fond 12 sensiblement plat et rond, et d'une paroi verticale circulaire 14. La base 10, comme le couvercle de la boite de Pétri non représenté, est en matériau plastique traditionnellement utilisé pour ce type de produit, tel que le polystyrène. Selon ce premier mode de réalisation, le fond 12 de la base 10 comporte en outre une nervure 16 continue et circulaire, faisant saillie et courant le long de la paroi verticale circulaire 14. Le rôle de cette nervure 16 est de retenir le milieu de culture gélosé disposé en couche dans la boite de Pétri. La nervure 16 joue donc un rôle de point d'ancrage du milieu de culture 30. Ceci est bien montré sur la figure 5.

Comme on peut le voir en détail sur la figure 3, la nervure circulaire 16 présente une section transversale de forme sensiblement triangulaire. En particulier, on constate que les côtés du triangle constituant les pentes de la nervure n'ont pas la même inclinaison. En effet, il est tout à fait judicieux que la pente 161 en regard de la paroi verticale circulaire 14 présente une inclinaison importante. Comme expliqué ci-dessus, la nervure 16 sert de point d'ancrage du milieu de culture. En effet, si de façon accessoire il peut empêcher le milieu de se décoller du fond, cet ancrage a pour vocation d'empêcher ledit milieu de culture de se rétracter. La rétraction se manifeste généralement par une réduction du diamètre de la couche de milieu de culture, de sorte que le bord de la couche du milieu de culture n'est plus en contact avec la face interne de la paroi verticale circulaire 14. La forte inclinaison de la pente 161 augmente le pouvoir de résistance à la rétraction de la nervure 16. Dans un autre mode de réalisation particulier, il est tout à fait envisageable que la pente 161 soit inversée, augmentant de ce fait le phénomène d'ancrage.

La hauteur de la nervure 16 doit être bien inférieure à l'épaisseur de la couche de milieu de culture. Ceci s'impose du fait qu'une couche trop fine de milieu de culture en aplomb de la nervure 16 génèrerait à cet endroit une zone de faiblesse du milieu, où ce dernier serait susceptible de se rompre en cas de contraintes mécaniques trop fortes et/ou répétées. Par ailleurs, la nervure ne doit pas gêner l'ensemencement du milieu de culture. Une nervure dont l'extrémité supérieure est proche de la surface du milieu de culture pourrait présenter un tel inconvénient, sans compter le risque de rupture, explicité ci-dessus, lors de l'application d'un outil d'ensemencement, tel qu'une oese. Tous ces inconvénients d'une nervure trop haute sont à mettre en balance avec les inconvénients d'une nervure dont la hauteur est trop faible, de sorte qu'elle ne joue plus son rôle d'ancrage.

Aussi, il est préférable que la hauteur de la nervure 16 soit préférentiellement comprise entre 0,15 et 5 millimètres (mm). Il est entendu que la valeur de hauteur est choisie en lien avec la forme de la section transversale de la nervure. Autrement dit, lorsque l'on souhaite disposer d'une nervure de faible hauteur, la diminution du pouvoir de retenue d'une nervure d'une telle hauteur peut être compensée par une forme de nervure améliorant la retenue, telle qu'une nervure présentant une pente 161 inversée.

Les figures 2 et 4 représentent la base 20 d'une boite de Pétri selon un deuxième mode de réalisation. Cette base 20 comporte comme la base 10, un fond sensiblement plan et rond 22 ainsi qu'une paroi verticale circulaire 24. Par contre, elle se distingue de la base 10 représentée sur les figures 1 et 3, par le fait qu'elle ne comporte pas une seule nervure continue, mais plusieurs nervures discontinues 26, en arc de cercle, courant le long de la paroi verticale circulaire 14. Ces nervures sont espacées les unes des autres d'un angle (constant ou non) compris entre 10 et 170 °. Il est à noter que la distance entre les nervures 26 prises deux par deux, peut également être variable. Concernant la longueur des nervures, elle correspond à un angle compris entre 10 et 170 °. Ainsi, pour une boite de Pétri de 90 mm de diamètre, cette distance est comprise entre 7 mm et 13 cm. De même, la longueur des nervures discontinues au sein d'une même boite de Pétri peut varier, d'une nervure à l'autre.

Comme on peut le voir sur la figure 4, ces nervures discontinues 26 présentent une section transversale de forme sensiblement identique à celle de la nervure 16, à savoir sensiblement triangulaire. On constate sur la figure 2 que les nervures discontinues 26 présentent des extrémités en pointe de flèches. En effet, la hauteur des nervures 26 diminue progressivement jusqu'à ce que la nervure disparaisse. Autrement dit, les trois arêtes des nervures 26 convergent à chacune des extrémités de ces dernières. Il est toutefois à noter que la forme desdites extrémités ne constitue qu'un mode particulier de réalisation et qu'on peut tout à fait envisager des nervures présentant des formes différentes.

La base 20 telle que présentée sur les figures 2 et 4 comporte au total 6 nervures. Il est nonobstant envisageable de disposer d'une base comportant moins ou plus de nervures discontinues. En effet, la base peut comporter par exemple deux nervures discontinues, dans ce cas, lesdites nervures seront de longueur plus importante. La base peut comporter par exemple 10 nervures, auquel cas ces dernières présentent une longueur moins importante que celles du mode de réalisation présenté en figure 2 et 4. Il importe dans tous les cas que la longueur totale de l'ensemble de nervures discontinues 26 soit au moins égale, voire supérieure à la moitié de la circonférence du cercle, sur le tracé duquel sont placées lesdites nervures discontinues 26.

La distance à laquelle se trouve les nervures 16 ou 26 de la paroi verticale circulaire 14 ou 24 respectivement est également un élément important à prendre en considération. En effet, le positionnement des nervures répond à plusieurs contraintes :
- En premier lieu, les nervures doivent être positionnées de telle manière qu'elles ne gênent pas l'analyse visuelle de la boite de Pétri ensemencée. En effet, l'identification des microorganismes passent encore par une analyse visuelle des colonies qui ont poussé sur le milieu de culture gélosé, qui donnent des informations importantes en termes d'orientation de l'analyse au microbiologiste. La présence des nervures ne doit donc surtout pas constituer un frein à une telle analyse visuelle ;
- En second lieu, les nervures doivent être positionnées de telle manière qu'elles jouent une rôle efficace contre la rétraction de la gélose. A cette fin, elles ne doivent être ni trop loin de la paroi verticale circulaire, auquel cas, leur action est limitée ; ni trop près de la paroi verticale circulaire, auquel cas le milieu de culture est fragilisé du fait que la quantité de matière entre les nervures et la paroi verticale circulaire est trop faible entraînant un risque de rupture du milieu de culture gélosé.

Aussi, avantageusement, la distance entre une nervure et la paroi verticale circulaire doit être comprise entre 0,5 et 15 mm.

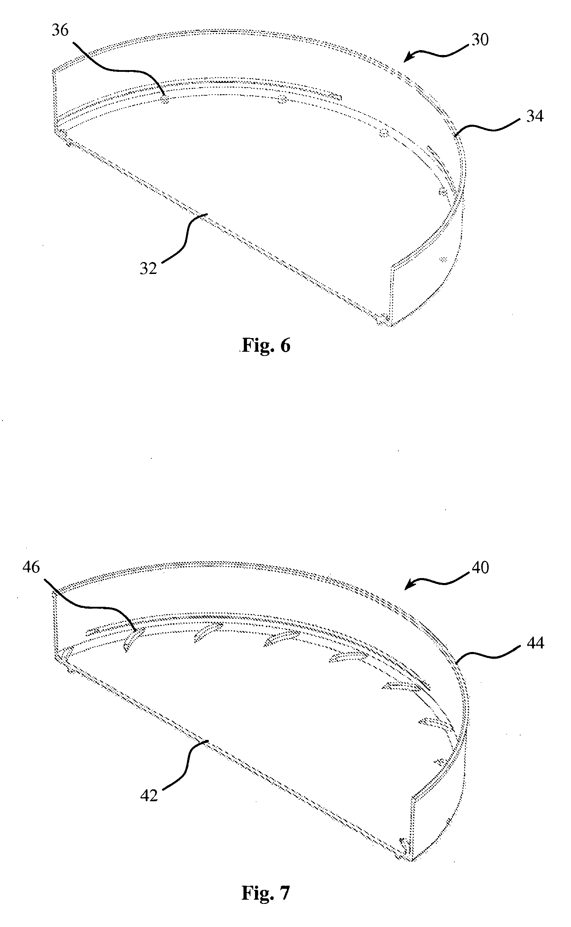

La figure 6 représente la base 30 d'une boite de Pétri selon un troisième mode de réalisation. Cette base 30 comporte un fond sensiblement plan et rond 32 ainsi qu'une paroi verticale circulaire 34. Par contre, elle se distingue des deux précédents modes de réalisation représentés respectivement sur les figures 1 et 3 et les figure 2 et 4, par le fait qu'elle ne comporte pas de nervures sur le fond 32, mais des plots cylindriques 36. Ces plots sont espacés les uns des autres d'un angle (constant ou non) compris entre 10 et 170 °. Il est à noter que la distance entre les plots 36 pris deux par deux, peut également être variable.

La figure 7 représente quant à elle un quatrième mode de réalisation sous la forme d'une base 40 d'une boite de Pétri. Cette base 40 comporte un fond sensiblement plan et rond 42 ainsi qu'une paroi verticale circulaire 44. Sur le fond 42 sont disposés des nervures en arc de cercle 46. Ces nervures 46 sont orientées sensiblement radialement. Ces plots sont espacés les uns des autres d'un angle (constant ou non) compris entre 10 et 170 °. Il est à noter que la distance entre les plots 36 pris deux par deux, peut également être variable. Le nombre et l'espacement des nervures peut varier, selon les variantes de ce mode de réalisation. A titre d'exemple, il est envisageable de disposer d'un nombre de nervure compris entre 4 et 20.

Selon un autre mode de réalisation particulier non représenté, la base de la boite de Pétri comporte plusieurs séries de nervures concentriques. Il peut s'agir, indépendamment d'une série de nervures à l'autre, de nervures continues ou discontinues. On peut ainsi envisager de disposer de plusieurs séries de nervures continues, de plusieurs séries de nervures discontinues ou d'un panachage de nervures continues et discontinues. Dans le cas où la base de la boite de Pétri, comporte plusieurs séries de nervures discontinues, ces dernières peuvent être de longueurs identiques ou différentes à l'intérieur d'une série ou entre deux séries différentes. De plus, les nervures discontinues peuvent être alignées radialement ou décalées les unes par rapport aux autres.

Selon un dernier mode de réalisation, la forme générale des nervures peut changer. En effet, les nervures selon les modes de réalisation présentés sur les figures ont une forme générale de cercle pour les nervures continues ou en arcs de cercle pour les nervures discontinues. On pourrait néanmoins disposer de nervures sous formes de segments de droite.

On peut également envisager une nervure continue ayant la forme générale d'une sinusoïde suivant la paroi verticale circulaire.

### EXEMPLE :

### Mesure de rétraction de milieux de culture gélosés dans des boites de Pétri comportant de moyens destinés à éviter la rétraction et des boites de Pétri classiques

Des mesures de rétraction d'un milieu de culture gélosé ont été réalisées avec deux types différents de boites de Pétri : une boite de Pétri classique et une boite de Pétri comportant des moyens permettant d'éviter la rétraction du milieu de culture gélosé. Lesdits moyens sont constitués par une série de 6 nervures discontinues; les nervures étant alignées radialement.

Le protocole de préparation du milieu de culture gélosé, de répartition de ce dernier dans les boites est identique pour toutes les boites de Pétri. Deux profils d'incubation différents ont été testés pour chaque type de boites.

### Essai n°1 :

- Production du milieu de culture gélosé et répartition dans les boites de Pétri :
   Le milieu de culture utilisé est un milieu bioMérieux ChromID® Ottaviani Agosti (Réf. 43641).
   16,5 kg de ce milieu sous forme sèche sont dilués dans de l'eau afin d'obtenir 228 litres (L) de milieu de culture. 720 boites de Pétri classiques et 720 boites avec nervures sont ainsi produites.
- Conditions de stockage/incubation des boites de Pétri :
   Les boites de Pétri classiques et les boites de Pétri avec nervures produites, tel qu'explicité ci-dessus, sont incubées dans les conditions suivantes :
   o Conditions de stockage réelles : 10-15°C pendant 4 jours (chambre de stockage en logistique), puis 2-8° .
   ∘ Conditions d'incubation classiques : 33-37°C pendant 2 jours (étuve)

### Essai n°2 :

- Production du milieu de culture gélosé et répartition dans les boites de Pétri :
   La même quantité de milieu bioMérieux ChromID® Ottaviani Agosti (Réf. 43641) est produite. On prépare ainsi 620 boites de Pétri classiques et 620 boites avec nervures.
- Conditions de stockage/incubation des boites de Pétri :
   Les boites de Pétri classiques et les boites de Pétri avec nervures produites tel qu'explicité ci-dessus ont été incubées dans les conditions suivantes :
   o Conditions de stockage réelles : 10-15°C pendant 5 jours (chambre de stockage en logistique)
   o Conditions d'incubation classiques : 33-37°C pendant 2 jours (étuve)

Par chacune des boites de Pétri produites dans les deux essais ci-dessus, une analyse visuelle de la rétraction du milieu de culture gélosé dans les boites après stockage/incubation est réalisée.

Le tableau 1 ci-dessous regroupe l'intégralité des résultats :

**Tableau 1**

| **ESSAIS** | **Essai n° 1** | **Essai n° 2** |
|---|---|---|
| Boite de Pétri classique | 124 boîtes rétractées sur 720 soit 17.2% | 120 boîtes rétractées sur 620 soit 19,3 % |
| Boite de Pétri avec nervures | 2 boîtes rétractées sur 720 soit 0,28% | Pas de rétraction |

Les 2 essais présentés ci-dessus montrent de manière très claire que les nervures présentes dans les boites de Pétri diminuent fortement, voire annulent le phénomène de rétraction du milieu de culture gélosé contenu dans les boites, et ce, quelles que soient les conditions de stockage ou d'incubation.

## Revendications

1. Boite de Pétri, destinée à recevoir un milieu de culture de microorganismes gélosé, comprenant une base recevant ledit milieu de culture et un couvercle apte à venir se positionner sur la base, ladite base étant constituée d'un fond sensiblement plat et rond et d'une paroi verticale cylindrique, ladite base et ledit couvercle étant sensiblement circulaires, ladite boite de Pétri étant **caractérisée en ce que** la base comprend au moins un moyen destiné à éviter la rétraction du milieu de culture, ledit moyen étant en saillie et positionné en périphérie du fond de la base.

2. Boite de Pétri selon la revendication 1 dans laquelle, le moyen destiné à éviter la rétraction du milieu de culture est constituée par au moins une nervure continue

3. Boite de Pétri selon la revendication 2, dans laquelle la nervure continue est circulaire ou sinusoïdale.

4. Boite de Pétri selon la revendication 1 dans laquelle le moyen destiné à éviter la rétraction du milieu de culture est constitué par au moins une nervure discontinue.

5. Boite de Pétri selon l'une des revendications précédentes, comprenant en outre des moyens de verrouillage du couvercle sur la base.

6. Boite de Pétri selon la revendication précédente, dans laquelle les moyens de verrouillage comprennent au moins une patte radiale et au moins une gaine radiale, positionnées respectivement soit sur la couvercle, soit sur la base, et coopérant pour verrouiller la boite de Pétri.

7. Boite de Pétri selon l'une des revendications 4 à 6, comprenant entre 2 et 12 nervures discontinues.

8. Boite de Pétri selon l'une des revendications 2 à 7, comprenant plusieurs séries de nervures concentriques, continues ou discontinues.

9. Boite de Pétri selon l'une des revendications précédentes, dans laquelle le ou les moyen(s) destiné(s) à éviter la rétraction du milieu de culture présente(nt) une hauteur comprise entre 0,15 et 5 millimètres.

10. Boite de Pétri selon l'une des revendications précédentes, dans laquelle le ou les moyens destiné à éviter la rétraction du milieu de culture sont situés à une distance comprise entre 0,5 et 15 millimètre du bord vertical circulaire de la base.

11. Procédé de préparation d'un milieu de culture gélosé comprenant les étapes consistant à :
- Disposer d'une boite de Pétri, selon l'une des revendications 1 à 10,
- Répartir un milieu de culture gélosé en fusion à l'intérieur de ladite boite de Pétri, de sorte que ce dernier recouvre l'intégralité du fond de la base,
- Laisser refroidir le milieu de culture gélosé de sorte que le ou les moyens destiné(s) à éviter la rétraction soient ancrés dans le milieu de culture gélosé.

## Patentansprüche

1. Petrischale, die dazu bestimmt ist, ein Agar-Nährmedium für Mikroorganismen aufzunehmen, die eine Grundplatte, die das Nährmedium aufnimmt, und einen Deckel enthält, der auf der Grundplatte positioniert werden kann, wobei die Grundplatte aus einem im Wesentlichen flachen und runden Boden und aus einer zylindrischen senkrechten Wand besteht, wobei die Grundplatte und der Deckel im Wesentlichen kreisförmig sind, wobei die Petrischale **dadurch gekennzeichnet ist, dass** die Grundplatte mindestens eine Einrichtung enthält, die dazu bestimmt ist, das Zurückziehen des Nährmediums zu vermeiden, wobei die Einrichtung vorsteht und am Umfang des Bodens der Grundplatte positioniert ist.

2. Petrischale nach Anspruch 1, wobei die Einrichtung zum Verhindern des Zurückziehens des Nährmediums aus mindestens einer durchgehenden Rippe besteht.

3. Petrischale nach Anspruch 2, wobei die durchgehende Rippe kreisförmig oder sinusförmig ist.

4. Petrischale nach Anspruch 1, wobei die zum Verhindern des Zurückziehens des Nährmediums bestimmte Einrichtung aus mindestens einer unterbrochenen Rippe besteht.

5. Petrischale nach einem der vorhergehenden Ansprüche, die außerdem Einrichtungen zur Verriegelung des Deckels an der Grundplatte enthält.

6. Petrischale nach dem vorhergehenden Anspruch, wobei die Verriegelungseinrichtungen mindestens eine radiale Lasche und mindestens eine radiale Umhüllung enthalten, die je auf dem Deckel bzw. auf der Grundplatte positioniert sind und zusammenwirken, um die Petrischale zu verriegeln.

7. Petrischale nach einem der Ansprüche 4 bis 6, die zwischen 2 und 12 unterbrochene Rippen enthält.

8. Petrischale nach einem der Ansprüche 2 bis 7, die mehrere Reihen von konzentrischen, durchgehenden oder unterbrochenen Rippen enthält.

9. Petrischale nach einem der vorhergehenden Ansprüche, wobei die zur Vermeidung des Zurückziehens des Nährmediums bestimmte(n) Einrichtung(en) eine Höhe zwischen 0,15 und 5 Millimeter aufweist (aufweisen).

10. Petrischale nach einem der vorhergehenden Ansprüche, wobei die zur Vermeidung des Zurückziehens des Nährmediums bestimmte(n) Einrichtung(en) sich in einem Abstand von zwischen 0,5 und 15 Millimeter vom kreisförmigen senkrechten Rand der Grundplatte befindet (befinden).

11. Verfahren zur Zubereitung eines Agar-Nährmediums, das die folgenden Schritte enthält:
- eine Petrischale nach einem der Ansprüche 1 bis 10 anzuordnen,
- ein schmelzflüssiges Agar-Nährmedium im Inneren der Petrischale so anzuordnen, dass es den ganzen Boden der Grundplatte bedeckt,
- das Agar-Nährmedium abkühlen zu lassen, damit die zur Vermeidung des Zurückziehens bestimmte(n) Einrichtung(en) im Agar-Nährmedium verankert wird (werden).

## Claims

1. Petri dish, intended for receiving an agar culture medium for microorganisms, comprising a base receiving said culture medium and a lid capable of being positioned on the base, said base being constituted of a substantially flat and round bottom and a cylindrical vertical wall, said base and said lid being substantially circular, said Petri dish being **characterised in that** the base includes at least one means intended to avoid the retraction of the culture medium, said means projecting and being positioned on the periphery of the bottom of the base.

2. Petri dish according to claim 1 wherein, the means intended to avoid the retraction of the culture medium is constituted by at least one continuous rib.

3. Petri dish according to claim 2, wherein the continuous rib is circular or sinusoidal.

4. Petri dish according to claim 1 wherein the means intended to avoid the retraction of the culture medium is constituted by at least one non-continuous rib.

5. Petri dish according to one of the preceding claims, further including means for locking the lid onto the base.

6. Petri dish according to the preceding claim, wherein the locking means include at least one radial fastening hook and at least one radial sheath, which are respectively positioned either on the lid, or on the base, and cooperate to lock the Petri dish.

7. Petri dish according to one of claims 4 to 6, including between 2 and 12 non-continuous ribs.

8. Petri dish according to one of claims 2 to 7, including several series of concentric, continuous and / or non-continuous ribs.

9. Petri dish according to one of the preceding claims, wherein the means which is (are) intended to avoid the retraction of the culture medium has (have) a height of between 0.15 and 5 mm.

10. Petri dish according to one of the preceding claims, wherein the means which is (are) intended to avoid the retraction of the culture medium is (are) situated at a distance of between 0.5 and 15 millimetres from the circular vertical edge of the base.

11. Method of preparing an agar culture medium comprising the steps consisting of:
- possessing a Petri dish, according to one of claims 1 to 10,
- distributing a molten agar culture medium inside said Petri dish, such that it covers the entire bottom of the base,
- allowing the agar culture medium to cool such that the means which is (are) intended to avoid the retraction are anchored in the agar culture medium.
